# EUROPÄISCHE PATENTSCHRIFT

(11) **EP 3 948 306 B1**
(45) Veröffentlichungstag und Bekanntmachung des Hinweises auf die Patenterteilung: **19.11.2025**
(21) Anmeldenummer: 20711091.7
(22) Anmeldetag: 06.03.2020
(51) Int. Cl.: G01R 1/36, G01R 15/08, G01R 19/00, H03F 3/45, H03F 3/72

(54) **VERSTÄRKERVORRICHTUNG ZUR VERSTÄRKUNG KLEINER STRÖME**
AMPLIFIER DEVICE FOR AMPLIFYING SMALL CURRENTS
DISPOSITIF D'AMPLIFICATION POUR L'AMPLIFICATION DE COURANTS FAIBLES

(30) Priorität: 29.03.2019 DE 102019108192
(43) Veröffentlichungstag der Anmeldung: 09.02.2022
(73) Patentinhaber: Inficon GmbH, 50968 Köln (DE)
(72) Erfinder: ROLFF, Norbert, 50169 Horrem (DE)
(74) Vertreter: Dompatent
(86) Internationale Anmeldenummer: PCT/EP2020/056031
(87) Internationale Veröffentlichungsnummer: WO 2020/200644

(56) Entgegenhaltungen:
- EP-B1- 0 615 669
- WO-A1-2011/008569
- WO-A2-2009/000236
- JP-A- 2010 085 384
- JP-A- 2017 198 661
- JP-A- H05 126 864
- US-A- 4 198 607
- US-B1- 9 806 685

## Beschreibung

Die Erfindung betrifft eine Vorrichtung und ein Verfahren zur Verstärkung kleiner Ströme.

In vielen Messgeräten, zum Beispiel in Massenspektrometern oder Vakuummessgeräten, erfolgt die Messwertbildung aufgrund von Strömen, die von positiv oder negativ geladenen, auf eine Elektrode auftreffenden Teilchen erzeugt werden. Der dynamische Bereich der auftretenden und zu messenden Ionen- und Elektronenstromstärken ist sehr groß. Er reicht häufig von einigen hundert Atto-Ampere bis zu einigen Mikro-Ampere und überstreicht damit mehrere Dekaden.

Insbesondere sind kleine Ströme bei Vakuummessgeräten, im unteren Druckbereich und bei Lecksuchgeräten zu messen. Dabei werden vielfach Massenspektrometer eingesetzt. Bei sehr kleinen Strömen unterhalb von 10 fA treten beim Aufbau eines Verstärkers, der zugleich auch größere Ströme messen kann, Schwierigkeiten bei der Schaltung auf, wenn eine Bereichsumschaltung von der Messung kleiner Ströme zur Messung größerer Ströme notwendig ist. Stromverstärker, die einen Widerstand verwenden, müssen für sehr kleine Ströme einen hochohmigen Widerstand beinhalten, da das Stromrauschen des Widerstands mit zunehmenden Werten abnimmt. Bei Strömen größer als zum Beispiel 10 pA kann der hochohmige Widerstand jedoch nicht verwendet werden, da der Spannungsabfall am Widerstand zu groß wird. Bei Kondensatoren als Referenzbauteil ergeben sich ähnliche Schwierigkeiten wie bei den Widerständen. Bei Verstärkern nach dem Stand der Technik werden Halbleiterschalter oder auch Relais zur Umschaltung von Widerständen oder Strompfaden eingesetzt, um ein Umschalten von einem Betriebszustand zur Messung kleiner Ströme hin zu einem Betriebszustand zur Messung größerer Ströme zu ermöglichen. In allen Fällen sind dafür zusätzliche Bauteile erforderlich, die an den empfindlichen Eingangsknoten, an dem kleinste Ströme fließen und gemessen werden müssen, angeschlossen werden.

Zur Messung derartiger Teilchenströme werden Messvorrichtungen eingesetzt, die aus dem Teilchenstrom, das heißt Ionenstrom oder Elektronenstrom, ein messbares Messpotenzial erzeugen. Die Messvorrichtung weist hierzu üblicherweise einen Stromverstärker auf, typischerweise mit einem Operationsverstärker. Über einen elektrischen Messwiderstand, der beispielsweise in dem Rücckopplungspfad des Operationsverstärkers angeordnet ist, wird aus dem verstärkten Strom ein messbares elektrisches Potenzial erzeugt.

Um mit derselben Messschaltung den gesamten Dynamikbereich der vorkommenden Teilchenströme abzudecken, ist sowohl bei den herkömmlichen als auch bei dem erfindungsgemäßen Verstärker vorgesehen, dass zwischen einem empfindlichen Betrieb zur Messung kleiner Ströme von weniger als 100 pA (z.B. 1 pA) und einem Betrieb zur Messung größerer Ströme von mindestens 1 pA (z.B. 100 pA) umgeschaltet wird. Bei den herkömmlichen Stromverstärkern erfolgt die Umschaltung mithilfe eines Relais, beispielsweise bei Sektorfeld-Massenspektrometern. Bei widerstandsbasierten Stromverstärkern, die einen großen Strombereich abdecken, kann auf eine Bereichsumschaltung nicht verzichtet werden, da bei kleinen Strömen nur hochohmige Widerstände infrage kommen und bei größeren Strömen nur niederohmige Bauteile den größeren Strom tragen können. Bei kleinen Strömen begrenzt das Widerstandsrauschen den Einsatzbereich.

In EP 0 615 669 B1 wird ein Verstärker beschrieben, bei dem das Stromsignal mittels Dioden übertragen wird. JP 2010 085384 A (OZAWA FUJIO) 15. April 2010 (2010-04-15) offenbart eine Strommessvorrichtung mit mithilfe von Schaltern umschaltbaren Messbereichen und einer Schutzvorrichtung am Eingang. WO 2009/000236 A2 (FORSCHUNGSZENTRUM JUELICH GMBH [DE] ET AL.) 31. Dezember 2008 (2008-12-31) offenbart eine Strommessvorrichtung mit automatisch umschaltbaren Messbereichen.

Der Erfindung liegt die Aufgabe zugrunde, eine Verstärkervorrichtung zum Verstärken kleiner Ströme zu schaffen, mit der das Umschalten zum Verstärken größerer Ströme verbessert wird, sowie ein entsprechendes Verfahren bereitzustellen.

Die erfindungsgemäße Verstärkervorrichtung ist definiert durch den unabhängigen Patentanspruch 1.

Erfindungsgemäß weist die Verstärkervorrichtung einen ersten Strompfad zur Verstärkung der kleinen Ströme auf. Der erste Strompfad enthält eine Eingangsverstärkervorrichtung mit mindestens einem ersten Verstärker mit mindestens einem Schutzelement, z.B. einer Schutzdiode, sowie einem Rückkoppelelement in einem den Ausgang der Eingangsverstärkervorrichtung mit dem invertierenden Eingang verbindenden Rückkopplungspfad. Zum Messen größerer Ströme ist ein zweiter, von dem ersten Strompfad zumindest teilweise verschieden ausgebildeter Strompfad vorgesehen und ausgebildet. Mindestens eines der Schutzelemente des ersten Verstärkers ist in dem zweiten Strompfad enthalten.

Somit wird erfindungsgemäß eine Verstärkervorrichtung bereitgestellt, an deren Eingang zur Messung größerer Ströme keine weiteren Bauteile als diejenigen zum Betrieb zur Messung kleinster Ströme erforderlich sind, nämlich der Stromeingang, der Messwiderstand in Form des Rückkoppelelements und die Eingangsverstärkervorrichtung mit mindestens dem ersten Verstärker. Die herkömmlicherweise verwendeten Eingangsverstärker weisen im Eingangsbereich bereits Schutzelemente, z.B. Schutzdioden auf, die in der integrierten Schaltung zum Schutz der Eingangskreise erforderlich sind. Diese Schutzelemente werden erfindungsgemäß zur Bereichsumschaltung verwendet. Damit ist der empfindlichste Bereich zur Messung kleinster Ströme der Schaltung so stabil, als ob keine weiteren Bereiche zur Messung anderer Stromgrößen vorhanden wären. Bei einem Bereichswechsel vom empfindlichen Bereich hin zu dem Bereich zur Messung größerer Ströme fließt der Eingangsstrom durch eine oder mehrere der Schutzelemente, wodurch der Eingangsbereich auch für deutlich größere Eingangsströme niederohmig wird. Da im integrierten Schaltkreis des ersten Verstärkers Schutzelemente vorhanden sein müssen, kann nicht jeder Verstärkerbaustein verwendet werden. Sowohl Verstärkerbausteine, die Schutzelemente zu den Versorgungsspannungen aufweisen, als auch Verstärkerbausteine, die über Schutzelemente zwischen den Eingangsleitungen verfügen, können verwendet werden.

Die Eingangsverstärkervorrichtung kann eine Anzahl von n Verstärkern mit n ≥ 1 aufweisen. Im einfachsten Fall von n = 1 beinhaltet die Eingangsverstärkervorrichtung nur den ersten Verstärker. Im Fall von n ≥ 2 kann die Eingangsverstärkervorrichtung aus mindestens zwei Verstärkern gebildet sein, die eine Verstärkerkombination bilden.

Die Eingangsverstärkervorrichtung weist einen invertierenden Eingang und einen Ausgang auf. Der Ausgang ist über einen Rückkopplungspfad mit dem invertierenden Eingang der Eingangsverstärkervorrichtung verbunden. In dem Rückkopplungspfad ist ein Rückkoppelelement enthalten.

Bei den Verstärkern kann es sich um Operationsverstärker und/oder bei den Schutzelementen um Schutzdioden handeln.

Im Folgenden werden anhand der Figuren Ausführungsbeispiele der Erfindung näher erläutert. Es zeigen:
- Fig. 1: das Ausführungsbeispiel in einem ersten Betriebszustand,
- Fig. 2: ein Detail aus Fig. 1,
- Fig. 3: das Ausführungsbeispiel in einem zweiten Betriebszustand und
- Fig. 4: ein Detail aus Fig. 1 bzw. Fig. 3 gemäß einem zweiten Ausführungsbeispiel.

Die Verstärkervorrichtung des dargestellten Ausführungsbeispiels weist einen ersten Operationsverstärker 1 auf, der einen invertierenden Eingang 30, einen nicht invertierenden Eingang 31, einen Ausgang 34, einen positiven Versorgungsanschluss 32 und einen negativen Versorgungsanschluss 33 aufweist.

Der Operationsverstärker beinhaltet Schutzelemente 18, 19, 20, 21 in diesem Fall Dioden die nach Art einer Brückengleichrichterschaltung zwischen dem invertierenden Eingang 30 und dem nicht invertierenden Eingang 31 sowie den beiden Versorgungsanschlüssen 32, 33 angeordnet sind und diese untereinander verbinden. Die Dioden 18, 19, 20, 21 sind dabei so angeordnet, dass sie vom negativen Versorgungsanschluss 33 in Richtung auf den positiven Versorgungsanschluss 32 leiten und in entgegengesetzter Richtung sperren.

Mit dem Ausdruck "nach Art einer Brückengleichrichterschaltung" ist gemeint, dass der Brückenzweig die beiden Eingänge 30, 31 des Operationsverstärkers 1 beinhaltet. Dem liegt der Gedanke zugrunde, dass bei einem idealen Operationsverstärker keine Spannung zwischen den beiden Eingängen 30, 31 abfällt. Das eine Ende des Brückenzweigs ist dabei mit dem invertierenden Eingang 30 und das andere Ende mit dem nicht invertierenden Eingang 31 verbunden. Die zwischen den beiden Eingängen anfallende Differenzspannung, die beim idealen Operationsverstärker gleich Null ist, ist damit Teil des Brückenzweigs.

Der Ausgang 34 des ersten Operationsverstärkers 1 ist mit dem nicht invertierenden Eingang 35 eines zweiten Operationsverstärkers 3 verbunden, dessen Ausgang über einen Rückkopplungszweig 41 mit dem invertierenden Eingang 30 des ersten Operationsverstärkers 1 verbunden ist. Der Rückkopplungspfad 41 enthält zwei Widerstände 2,4, zwischen denen der Rückkopplungspfad 41 über einen dritten Schalter 12 mit einem Eingang 22 verbunden ist, an dem eine Spannung einer Diodenflussspannung von ca. 0,5 Volt liegt.

Der Ausgang 16 des zweiten Operationsverstärkers 3 ist ferner über Regelelemente 5 mit Masse verbunden. Die Regelelemente 5 bestehen aus einem Widerstand 5a, einem Kondensator 5b und einem weiteren Widerstand 5c. Zwischen dem Kondensator 5b und dem weiteren Widerstand 5c ist der invertierende Eingang 36 des zweiten Operationsverstärkers 3 zu dessen Rückkopplung elektrisch angeschlossen.

Der Ausgang 34 des ersten Operationsverstärkers 1 ist über einen ersten Widerstand 6a mit dem positiven Versorgungsanschluss 32 des ersten Operationsverstärkers 1 verbunden und über einen zweiten Widerstand 6b mit dem negativen Versorgungsanschluss 33 verbunden. Der negative Versorgungsanschluss 33 ist über einen ersten Schalter 10 abwechselnd mit einer an der Spannungsquelle 14 anliegenden Versorgungsspannung von -5 Volt oder mit Masse verbindbar.

Der positive Versorgungsanschluss 32 des ersten Operationsverstärkers 1 ist elektrisch mit dem invertierenden Eingang 38 eines dritten Operationsverstärkers 7 verbunden, dessen Ausgang 17 über einen Widerstand 8 rückgekoppelt ist. Der Widerstand 8 ist dabei durch eine Diode 9 überbrückt, die in Richtung von dem Ausgang 17 des dritten Operationsverstärkers 7 auf den positiven Versorgungsanschluss 32 des ersten Operationsverstärkers 1 leitet und in entgegengesetzter Richtung sperrt.

Der erste Verstärker 1 und der zweite Verstärker 3 bilden eine Eingangsverstärkervorrichtung 50, wobei die beiden Verstärker 1, 3 eine Verstärkerkombination bilden. Der Rückkopplungspfad 41 verbindet den Ausgang der Eingangsverstärkerkombination 50 mit deren invertierendem Eingang 30.

Der nicht invertierende Eingang 37 des dritten Operationsverstärkers 7 ist über einen zweiten elektrischen Schalter 11 wahlweise mit einer Spannungsquelle 15, im vorliegenden Fall 5 Volt, oder mit Masse verbindbar.

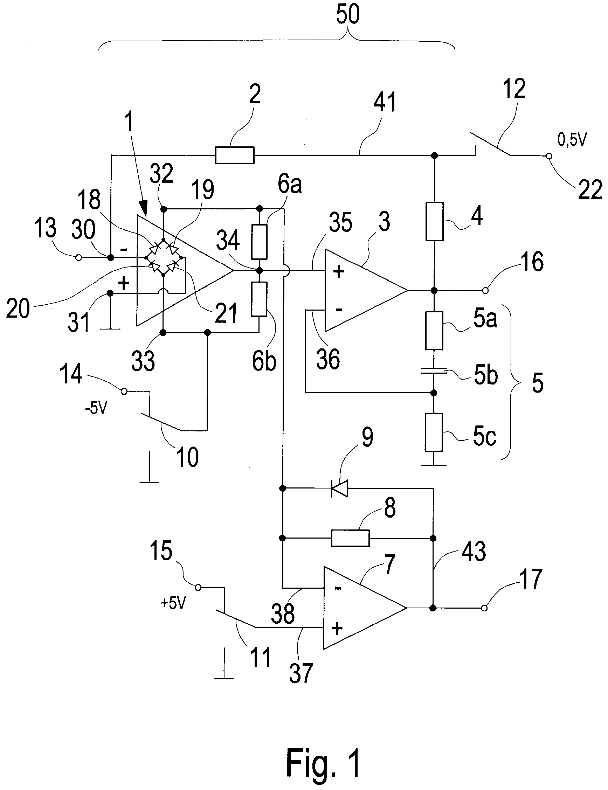

Bei dem dargestellten Ausführungsbeispiel der erfindungsgemäßen Verstärkervorrichtung wird als Eingangsverstärker der erste Operationsverstärker 1 verwendet, dessen Schutzelemente 18, 19, 20, 21 als Eingangsschutzdioden zur Versorgungsspannung vorgesehen sind. Wenn die Verstärkervorrichtung zur Messung kleiner Ströme von z.B. weniger als 100 pA im empfindlichsten Bereich betrieben wird, gelangt das Eingangsstromsignal über den Eingang 13 zum ersten Operationsverstärker 1. Die negative Versorgungspannung erhält der erste Operationsverstärker 1 aus der Spannungsquelle 14 über dem ersten Schalter 10 in der in Fig. 1 dargestellten Schalterstellung. Die positive Versorgungsspannung erhält der Operationsverstärker 1 aus der Spannungsquelle 15, im vorliegenden Fall in Höhe von +5 Volt, über den zweiten Schalter 11 in der in Fig. 1 dargestellten Schalterstellung am nicht invertierenden Eingang 37 des dritten Operationsverstärkers 7.

Die Ausgangsspannung am Ausgang 17 des dritten Operationsverstärkers 7 regelt sich auf eine um die Diodenflussspannung der Diode 9 erhöhten Wert ein. Dadurch wird die Spannung, die zum ersten Operationsverstärker 1 gelangt, so groß wie die Spannung am nicht invertierenden Eingang 37 des dritten Operationsverstärkers 7.

Der Verstärker 7 ist nicht Teil der Eingangsverstärkervorrichtung 50. Der Verstärker 7 dient zur Aufnahme größerer Ströme am Eingang 13 der Eingangsverstärkervorrichtung 50. Der Eingangsstrom fließt über die Diode 18 durch den Verstärker 1 direkt zum Eingang 38 des Verstärkers 7. Kleinere Ströme am Eingang 13 werden hingegen zunächst von der Eingangsverstärkervorrichtung 50 verstärkt.

Der dritte elektrische Schalter 12 ist in diesem in Fig. 1 dargestellten Betriebszustand zur Messung kleiner Ströme geöffnet. Die Rückkopplungswiderstände 4, 2 ergeben zusammen das Rückkoppelelement, im vorliegenden Fall in Form eines elektrischen Widerstands.

Der Eingang 13 am invertierenden Eingang 30 des ersten Operationsverstärkers 1 stellt in diesem Fall einen virtuellen Nullpunkt dar, wodurch am Eingang 13 keine nennenswerte Spannung anliegt.

Am Ausgang 16 des zweiten Operationsverstärkers 3 liegt eine zum Eingangsstrom des ersten Operationsverstärkers 1 proportionale invertierte Spannung an. Die Schutzdioden 18, 19, 20, 21 des Eingangsverstärkers werden nun jeweils in Sperrrichtung betrieben und führen somit keinen nennenswerten Strom.

Die Eingangsverstärkervorrichtung 50 aus dem ersten Operationsverstärker 1 und dem zweiten Operationsverstärker 3 bildet einen Regelkreis. Die Regelelemente 5 müssen je nach Grenzfrequenz der eingesetzten Elemente 5a, 5b, 5c dimensioniert werden, damit der Kreis stabil bleibt, zum Beispiel 120 kOhm am elektrischen Widerstand 5a, 1 nF am Kondensator 5b und 10 kOhm am elektrischen Widerstand 5c.

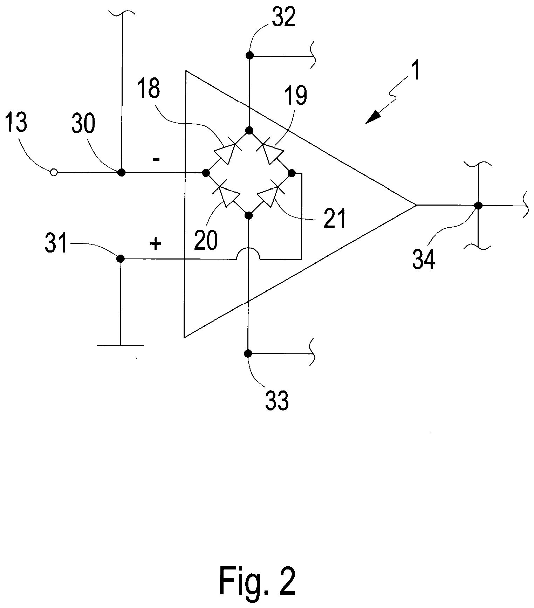

Fig. 1 zeigt die gesamte Verstärkervorrichtung, während Fig. 2 den ersten Operationsverstärker 1 und die darin angeordneten Schutzelemente 18, 19, 20, 21 und deren elektrische Verschaltung mit den Anschlüssen des ersten Operationsverstärkers 1 im Detail darstellt.

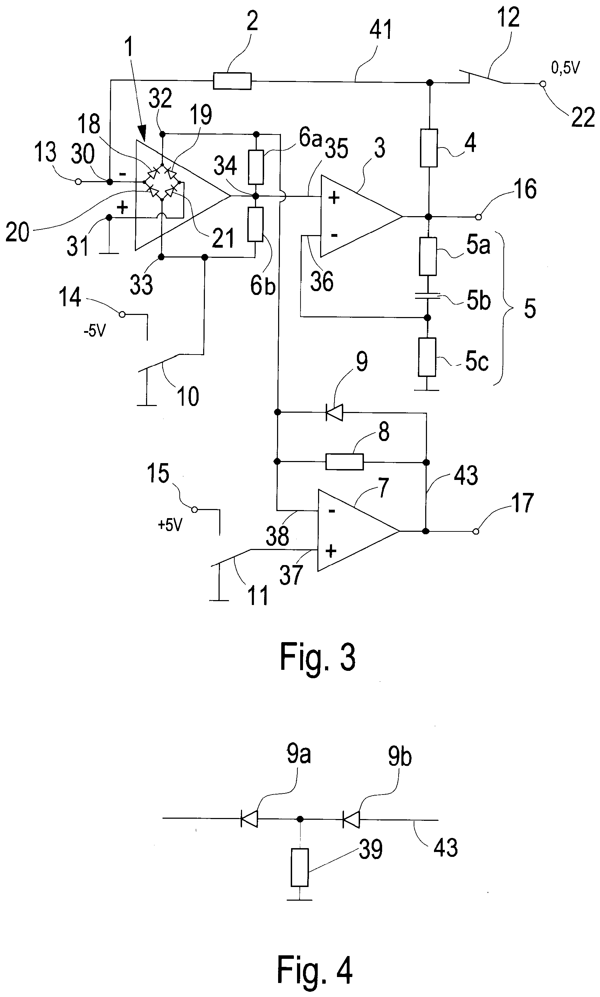

Fig. 3 zeigt die Verstärkervorrichtung im Betriebszustand zur Messung größerer Ströme über den zweiten Strompfad. Hierzu wird die Verstärkervorrichtung in einem erweiterten Strombereich betrieben, in dem der erste elektrische Schalter 10 auf Massepotenzial umgeschaltet wird. Damit liegt am negativen Versorgungsanschluss 33 des Eingangsverstärkers eine Spannung von ungefähr 0 Volt an. Die Eingangsschutzelemente 20, 21 sperren weiterhin.

Der zweite Schalter 11 wird ebenfalls auf Massepotenzial umgelegt. Da sich der dritte Operationsverstärker 7 mit dessen Rückkoppelwiderstand 8 so regelt, dass keine nennenswerte Differenzspannung zwischen dem invertierenden Eingang 38 und dem nicht invertierenden Eingang 37 des dritten Operationsverstärkers 7 anliegt, wird auch die positive Versorgung des ersten Operationsverstärkers 1 am positiven Versorgungsanschluss 32 nahe 0 Volt liegen. Wenn nun ein Eingangsstrom in den Eingangsanschluss 13 gelangt, fließt dieser durch die Schutzdiode 18 hin zum positiven Versorgungsanschluss 32.

Der dritte elektrische Schalter 12 ist in diesem Betriebszustand geschlossen, sodass die an der Klemme 22 anliegende Spannung, im vorliegenden Fall 0,5 Volt, an dem Rückkopplungswiderstand 2 entsprechend der Diodenflussspannung der Schutzdiode 18 anliegt, damit durch den Rückkoppelwiderstand 2 möglichst kein Strom fließt.

Der zweite Strompfad für größere Ströme am Eingang 13 führt nun also über die Schutzdiode 18 hin zum Rückkoppelwiderstand 8 des dritten Operationsverstärkers 7. Über dem Rückkopplungswiderstand 8 liegt eine dem Eingangsstrom proportionale invertierte Spannung an, die am Ausgang 17 des dritten Operationsverstärkers 7 abgegriffen werden kann. Die Diode 9 sperrt in diesem Fall. Der zweite Strompfad zur Messung größerer Ströme führt dann von dem invertierenden Eingang 30 des ersten Operationsverstärkers 1 über eine Schutzdiode 18 der Schutzdioden 18, 19, 20, 21 hin zum positiven Versorgungsanschluss 32 des ersten Operationsverstärkers 1 und von dort über den Rückkoppelwiderstand 8 des dritten Operationsverstärkers 7 hin zu dessen Ausgangsanschluss 17.

In der beschriebenen Schaltungsanordnung wird die Eingangsspannung 13 beim Betrieb im Strompfad für größere Ströme um die Flussspannung des Schutzelements 18 erhöht. Zur Vermeidung dieser Eigenschaft, können die Massepunkte an den Schalter 10 und 11 um die Flussspannung des Schutzelements 18 gesenkt werden. Das Rückkoppelelement 2 wird in diesem Fall stromlos, wenn am Eingang 220 Volt angelegt werden.

Eine weitere Verbesserung des zweiten Strompfades ergibt sich, wenn die Diode 9 nach Fig. 4 aufgeteilt wird. Im Fall, wenn der zweite Strompfad aktiviert wurde, liegt am Ausgang 17 eine negative Spannung an. Der Sperrstrom der Diode verfälscht die Messung. Wenn die Schaltung nach Fig. 4 ergänzt wird, fällt die negative Spannung an der Diode 9b ab. An der Diode 9a liegt dann beidseitig etwa null Volt an, wodurch sich der Sperrstrom deutlich reduziert.

Hier wurde die Schaltung für positive Eingangsströme beschrieben. Grundsätzlich ist eine Schaltungsvariante für negative bzw. auch beide Stromrichtungen möglich.

## Patentansprüche

1. Verstärkervorrichtung zur Aufnahme von Strömen, mit
einem ersten Strompfad zur Messung kleiner Ströme von weniger als 100 pA,
einer in dem ersten Strompfad enthaltenen Eingangsverstärkervorrichtung (50) mit
mindestens einem ersten Verstärker (1),
einem Ausgang,
einem invertierenden Eingang (30),
einem nicht-invertierenden Eingang (31)
einem den Ausgang mit dem invertierenden Eingang (30) verbindenden ersten Rückkopplungspfad (41) und
einem in dem ersten Rückkopplungspfad (41) enthaltenen Rückkoppelelement (2),
wobei der erste Verstärker (1) mindestens ein Schutzelement (18, 19, 20, 21) aufweist,
einem zweiten Strompfad zur Messung größerer Ströme mit einem Maximalstrom von mindestens dem 10-fachen des im ersten Strompfad aufzunehmenden Stromes,
**dadurch gekennzeichnet,**
**dass** mindestens eins der Schutzelemente (18, 19, 20, 21) der Eingangsverstärkervorrichtung (50) Teil des zweiten Strompfades ist, so dass bei einem Wechsel des Messbereichs von der Messung kleiner Ströme im ersten Strompfad zu der Messung großer Ströme im zweiten Strompfad der Eingangsstrom durch das mindestens eine Schutzelement fließt, wobei zur Umschaltung zwischen dem ersten und dem zweiten Strompfad ein negativer Versorgungsanschluss (33) des ersten Verstärkers (1) über einen ersten Schalter (10) abwechselnd mit einer an einer Spannungsquelle (14) anliegenden negativen Versorgungsspannung oder mit Masse verbindbar ist.

2. Verstärkervorrichtung nach Anspruch 1, **dadurch gekennzeichnet, dass** an mindestens einem der Schutzelemente (18, 19, 20, 21) die Versorgungsspannung des ersten Verstärkers (1) anliegt.

3. Verstärkervorrichtung nach einem der vorhergehenden Ansprüche, **dadurch gekennzeichnet, dass** mindestens eins der Schutzelemente (18, 19, 20, 21) zwischen zwei Eingängen (30, 31) des ersten Verstärkers (1) angeordnet und mit diesem verbunden ist.

4. Verstärkervorrichtung nach einem der vorhergehenden Ansprüche, **dadurch gekennzeichnet, dass** das Rückkoppelelement (2) im ersten Rückkopplungspfad (41) ein elektrischer Widerstand ist.

5. Verstärkervorrichtung nach einem der vorhergehenden Ansprüche, **dadurch gekennzeichnet, dass** das Rückkoppelbauteil (2) im ersten Rückkopplungspfad (41) ein Kondensator ist.

6. Verstärkervorrichtung nach einem der vorhergehenden Ansprüche, **dadurch gekennzeichnet, dass** die Eingangsverstärkervorrichtung (50) mindestens einen weiteren Verstärker (3) aufweist, der mit dem ersten Verstärker (1) einen Regelkreis bildet.

7. Verstärkervorrichtung nach einem der vorhergehenden Ansprüche, **dadurch gekennzeichnet, dass** mindestens einer der beiden Versorgungsanschlüsse (32, 33) des ersten Verstärkers (1) über einen elektrischen Widerstand (6a, 6b) mit dem Ausgang des ersten Verstärkers (1) verbunden ist.

8. Verstärkervorrichtung nach einem der vorhergehenden Ansprüche, **dadurch gekennzeichnet, dass** der Rückkopplungspfad (41) den Ausgang des letzten Verstärkers der Eingangsverstärkervorrichtung (50) bestehend aus dem ersten Verstärker (1) sowie dem mindestens einen weiteren Verstärker (3) mit dem invertierenden Eingang (30) der Eingangsverstärkervorrichtung (50) verbindet.

9. Verstärkervorrichtung nach einem der vorhergehenden Ansprüche, **dadurch gekennzeichnet, dass** der negative Versorgungsanschluss (33) des ersten Verstärkers (1) über einen ersten Schalter (10) wahlweise mit einer negativen Versorgungsspannung (14) oder Masse verbindbar ist, während der positive Versorgungsanschluss (32) des ersten Verstärkers (1) mit dem invertierenden Anschluss (38) eines dritten Verstärkers (7) elektrisch leitend verbunden ist, dessen nicht invertierender Eingang (37) über einen zweiten Schalter (11) wahlweise mit einer positiven Versorgungsspannung (15) oder Masse verbindbar ist.

10. Verstärkervorrichtung nach dem vorhergehenden Anspruch, **dadurch gekennzeichnet, dass** der dritte Verstärker (7) einen zweiten Rückkopplungspfad (43) mit einem elektrischen Widerstand (8) und einer parallel zu dem Widerstand (8) angeordneten elektrischen Diode (9) aufweist, die in Richtung von dem Ausgang (17) des dritten Verstärkers zu dem positiven Versorgungsanschluss (32) des ersten Verstärkers (1) leitet und in entgegengesetzter Richtung sperrt.

11. Verstärkervorrichtung nach einem der vorhergehenden Ansprüche, **dadurch gekennzeichnet, dass** der erste Rückkopplungspfad (41) über einen dritten Schalter (12) wahlweise mit einer Spannung verbindbar ist, die den Stromfluss im Rückkoppelelement (2) je nach Schalterstellung vermeidet oder erlaubt.

12. Verstärkervorrichtung nach einem der vorhergehenden Ansprüche, **dadurch gekennzeichnet, dass** die Massebezüge der Schalter (10, 11) sowie die Bezugsspannung (22) um die Flussspannung des Schutzelements (18) verändert wird.

13. Verstärkervorrichtung nach einem der vorhergehenden Ansprüche, **dadurch gekennzeichnet, dass** der zweite Rückkopplungspfad (43) zwei Dioden (9a, 9b) aufweist, zwischen denen ein Widerstand (39) mit Bezug zum Schalter (11) führt.

14. Gasdetektor mit einem massenspektrometrischen Sensor oder einem Totaldrucksensor und mit einer Verstärkervorrichtung nach einem der vorhergehenden Ansprüche.

15. Verfahren zum Messen von Strömen unter Verwendung einer Verstärkervorrichtung nach einem der Ansprüche 1 - 13, **dadurch gekennzeichnet, dass** eine Umschaltung von dem ersten Strompfad auf den zweiten Strompfad erfolgt, indem der negative Versorgungsanschluss (33) des ersten Verstärkers (1) über den ersten Schalter (10) mit Masse verbunden wird.

16. Verfahren nach einem der vorhergehenden Ansprüche, **dadurch gekennzeichnet, dass** zum Umschalten des Strompfades zusätzlich der nicht invertierende Eingang eines dritten Verstärkers (7), dessen invertierender Eingang mit dem positiven Versorgungsanschluss (32) des ersten Verstärkers (1) verbunden ist, mit Masse verbunden wird.

## Claims

1. An amplifier device for receiving currents, comprising
a first current path for measuring smaller currents of less than 100 pA,
an input amplifier device (50) included in the first current path, comprising
at least one first amplifier (1),
an output,
an inverting input (30),
a non-inverting input (31),
a first feedback path (41) connecting the output to the inverting input (30), and
a feedback element (2) included in the first feedback path (41),
wherein the first amplifier (1) has at least one protective element (18, 19, 20, 21),
a second current path for measuring larger currents with a maximum current of at least 10 times the current to be received in the first current path,
**characterized in that,**
at least one of the protective elements (18, 19, 20, 21) of the input amplifier device (50) is part of the second current path so that when changing the measuring area from the measurement of small currents in the first current path to the measurement of large currents in the second current path, the input current flows through the at least one protective element, wherein a negative supply connection (33) of the first amplifier (1) is alternately connectible via first switch (10) to a negative supply voltage applied to a voltage source (14) or to ground for switching between the first and the second current path.

2. The amplifier device according to claim 1, **characterized in that** the supply voltage of the first amplifier (1) is applied to at least one of the protective elements (18, 19, 20, 21).

3. The amplifier device according to any one of the preceding claims, **characterized in that** at least one of the protective elements (18, 19, 20, 21) is arranged between two inputs (30, 31) of the first amplifier (1) and connected thereto.

4. The amplifier device according to any one of the preceding claims, **characterized in that** the feedback element (2) in the first feedback path (41) is an electrical resistor.

5. The amplifier device according to any one of the preceding claims, **characterized in that** the feedback component (2) in the first feedback path (41) is a capacitor.

6. The amplifier device according to any one of the preceding claims, **characterized in that** the input amplifier device (50) comprises at least one further amplifier (3) forming a control circuit with the first amplifier (1).

7. The amplifier device according to any one of the preceding claims, **characterized in that** at least one of the two supply connections (32, 33) of the first amplifier (1) is connected via an electrical resistor (6a, 6b) to the output of the first amplifier (1).

8. The amplifier device according to any one of the preceding claims, **characterized in that** the feedback path (41) connects the output of the last amplifier of the input amplifier device (50) consisting of the first amplifier (1) and the at least one further amplifier (3) to the inverting input (30) of the input amplifier device (50).

9. The amplifier device according to any one of the preceding claims, **characterized in that** the negative supply connection (33) of the first amplifier (1) is selectively connectible via a first switch (10) to a negative supply voltage (14) or to ground, while the positive supply connection (32) of the first amplifier (1) is connected in an electrically conductive manner to the inverting connection (38) of a third amplifier (7), the non-inverting input (37) of which is selectively connectible via a second switch (11) to a positive supply voltage (15) or to ground.

10. The amplifier device according to any one of the preceding claims, **characterized in that** the third amplifier (7) comprises a second feedback path (43) with an electrical resistor (8) and an electric diode (9) arranged in parallel with the resistor (8) and conducting in the direction from the output (17) of the third amplifier to the positive supply connection (32) of the first amplifier (1) and blocking in the opposite direction.

11. The amplifier device according to any one of the preceding claims, **characterized in that** the first feedback path (41) is selectively connectible via a third switch (12) to a voltage which avoids or allows the current flow in the feedback element (2) depending on the switch position.

12. The amplifier device according to any one of the preceding claims, **characterized in that** the ground references of the switches (10, 11) and the reference voltage (22) are changed by the forward voltage of the protective element (18).

13. The amplifier device according to any one of the preceding claims, **characterized in that** the second feedback path (43) comprises two diodes (9a, 9b) between which a resistor (39) leads with respect to the switch (11).

14. A gas detector comprising a mass spectrometric sensor or a total pressure sensor and an amplifier device according to any one of the preceding claims.

15. A method for measuring currents by using an amplifier device according to any one of claims 1-13, **characterized in that** switching from the first current path to the second current path takes place by connecting the negative supply connection (33) of the first amplifier (1) via the first switch (10) to ground.

16. The method according to any one of the preceding claims, **characterized in that**, for switching the current path, the non-inverting input of a third amplifier (7), whose inverting input is connected to the positive supply connection (32) of the first amplifier (1), is additionally connected to ground.

## Revendications

1. Dispositif d'amplification permettant d'absorber des courants, comprenant
un premier chemin de courant permettant de mesurer des petits courants inférieurs à 100 pA,
un dispositif d'amplification d'entrée (50) contenu dans le premier chemin de courant et comprenant
au moins un premier amplificateur (1),
une sortie,
une entrée inverseuse (30),
une entrée non inverseuse (31)
un premier chemin de rétroaction (41) connectant la sortie à l'entrée inverseuse (30) et
un élément de rétroaction (2) contenu dans le premier chemin de rétroaction (41),
le premier amplificateur (1) comprenant au moins un élément de protection (18, 19, 20, 21),
un deuxième chemin de courant permettant de mesurer des courants plus importants avec un courant maximal d'au moins 10 fois le courant à absorber dans le premier chemin de courant,
**caractérisé en ce**
**qu'**au moins un des éléments de protection (18, 19, 20, 21) du dispositif d'amplification d'entrée (50) fait partie du deuxième chemin de courant, de sorte que, lors d'un changement de la plage de mesure lors du passage de la mesure de petits courants dans le premier chemin de courant à la mesure de courants importants dans le deuxième chemin de courant, le courant d'entrée passe par ledit au moins un élément de protection, une borne d'alimentation négative (33) du premier amplificateur (1) pouvant être connectée de manière alternée à la masse ou à une tension d'alimentation négative appliquée à une source de tension (14) en vue d'une commutation entre les premier et deuxième chemins de courant par l'intermédiaire d'un premier commutateur (10).

2. Dispositif d'amplification selon la revendication 1, **caractérisé en ce que** la tension d'alimentation du premier amplificateur (1) est appliquée à au moins un des éléments de protection (18, 19, 20, 21).

3. Dispositif d'amplification selon l'une quelconque des revendications précédentes, **caractérisé en ce qu'**au moins un des éléments de protection (18, 19, 20, 21) est agencé entre deux entrées (30, 31) du premier amplificateur (1) et est connecté audit premier amplificateur.

4. Dispositif d'amplification selon l'une quelconque des revendications précédentes, **caractérisé en ce que** l'élément de rétroaction (2) dans le premier chemin de rétroaction (41) est une résistance électrique.

5. Dispositif d'amplification selon l'une quelconque des revendications précédentes, **caractérisé en ce que** l'élément de rétroaction (2) dans le premier chemin de rétroaction (41) est un condensateur.

6. Dispositif d'amplification selon l'une quelconque des revendications précédentes, **caractérisé en ce que** le dispositif d'amplification d'entrée (50) compend au moins un autre amplificateur (3) qui forme un circuit de régulation avec le premier amplificateur (1).

7. Dispositif d'amplification selon l'une quelconque des revendications précédentes, **caractérisé en ce qu'**au moins une des deux bornes d'alimentation (32, 33) du premier amplificateur (1) est connectée à la sortie du premier amplificateur (1) par l'intermédiaire d'une résistance électrique (6a, 6b).

8. Dispositif d'amplification selon l'une quelconque des revendications précédentes, **caractérisé en ce que** le chemin de rétroaction (41) connecte la sortie du dernier amplificateur du dispositif d'amplification d'entrée (50), composé du premier amplificateur (1) et dudit au moins un autre amplificateur (3), à l'entrée inverseuse (30) du dispositif d'amplification d'entrée (50).

9. Dispositif d'amplification selon l'une quelconque des revendications précédentes, **caractérisé en ce que** la borne d'alimentation négative (33) du premier amplificateur (1) peut être connectée au choix à une tension d'alimentation négative (14) ou à une masse par l'intermédiaire d'un premier commutateur (10), tandis que la borne d'alimentation positive (32) du premier amplificateur (1) est connectée de manière électriquement conductrice à la borne inverseuse (38) d'un troisième amplificateur (7) dont l'entrée non inverseuse (37) peut être connectée au choix à une tension d'alimentation positive (15) ou à une masse par l'intermédiaire d'un deuxième commutateur (11).

10. Dispositif d'amplification selon la revendication précédente, **caractérisé en ce que** le troisième amplificateur (7) comprend un deuxième chemin de rétroaction (43) avec une résistance électrique (8) et une diode électrique (9) qui est agencée parallèlement à la résistance (8) et qui conduit dans une direction allant de la sortie (17) du troisième amplificateur à la borne d'alimentation positive (32) du premier amplificateur (1) et bloque dans la direction opposée.

11. Dispositif d'amplification selon l'une quelconque des revendications précédentes, **caractérisé en ce que** le premier chemin de rétroaction (41) peut être connecté au choix par l'intermédiaire d'un troisième commutateur (12) à une tension qui empêche ou autorise le flux de courant dans l'élément de rétroaction (2) en fonction de la position de commutateur.

12. Dispositif d'amplification selon l'une quelconque des revendications précédentes, **caractérisé en ce que** les références de masse des commutateurs (10, 11) ainsi que la tension de référence (22) sont modifiées par la tension directe de l'élément de protection (18).

13. Dispositif d'amplification selon l'une quelconque des revendications précédentes, **caractérisé en ce que** le deuxième chemin de rétroaction (43) comprend deux diodes (9a, 9b) entre lesquelles est connectée une résistance (39) liée au commutateur (11).

14. Détecteur de gaz comprenant un capteur de spectrométrie de masse ou un capteur de pression totale et comprenant un dispositif d'amplification selon l'une quelconque des revendications précédentes.

15. Procédé de mesure de courants à l'aide d'un dispositif d'amplification selon l'une quelconque des revendications 1 à 13, **caractérisé en ce qu'**une commutation du premier chemin de courant au deuxième chemin de courant est effectuée en connectant la borne d'alimentation négative (33) du premier amplificateur (1) à la masse par l'intermédiaire du premier commutateur (10).

16. Procédé selon l'une quelconque des revendications précédentes, **caractérisé en ce que**, afin de commuter le chemin de courant, l'entrée non inverseuse d'un troisième amplificateur (7), dont l'entrée inverseuse est connectée à la borne d'alimentation positive (32) du premier amplificateur (1), est en outre connectée à la masse.
